(19) Europäisches Patentamt
European Patent Office
Office européen des brevets

(11) Publication number: **0 071 543 B1**

(12) **EUROPEAN PATENT SPECIFICATION**

(45) Date of publication of patent specification: **21.10.87**

(51) Int. Cl.$^4$: **C 07 D 473/10**, A 61 K 31/52 // C07C149/34, C07D295/08

(21) Application number: **82401446.8**

(22) Date of filing: **30.07.82**

(54) **Piperazine derivatives of theobromine.**

(30) Priority: **31.07.81 US 288847**

(43) Date of publication of application: **09.02.83 Bulletin 83/06**

(45) Publication of the grant of the patent: **21.10.87 Bulletin 87/43**

(84) Designated Contracting States: **AT BE CH DE FR GB IT LI LU NL SE**

(56) References cited:
**EP-A-0 005 385**
**EP-A-0 033 674**
**DE-A-1 966 620**
**FR-A-2 450 831**
**GB-A-1 133 989**
**GB-A-1 289 287**

**The file contains technical information submitted after the application was filed and not included in this specification**

(73) Proprietor: **LABORATOIRES SYNTEX**
**20 rue Jean Jaurès**
**F-92807 Puteaux, Hauts de Seine (FR)**

(72) Inventor: **Favier, Colette**
**21 Avenue de Bretteville**
**F-92200 Neuilly-sur-Seine (FR)**
Inventor: **Pinhas, Henri**
**39 Rue Truffaut**
**F-75017 Paris (FR)**
Inventor: **Beranger, Serge**
**12 Allée des Cèdres**
**F-91220 Bretigny-sur-Orge (FR)**
Inventor: **Pascal, Jean-Claude**
**23-25 Allée des Hautes Bruyères**
**F-94230 Cachan (FR)**

(74) Representative: **Polus, Camille et al**
**c/o Cabinet Lavoix 2, Place d'Estienne d'Orves**
**F-75441 Paris Cedex 09 (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 071 543 B1

Courier Press, Leamington Spa, England.

**Description**

BACKGROUND OF THE INVENTION

This invention relates to piperazine derivatives of theobromine and to their utility as treatment compounds for respiratory and allergic diseases.

Theobromine, itself, is well known as a diuretic, cardiac stimulant and smooth muscle relaxant and vasodilator. Addition of the piperazine containing substituent confers a range of pharmacologic activities which render the resulting compounds useful in the symptomatic treatment of asthma, hay fever and other respiratory diseases such as, for example, the common cold. The compounds of the present invention are also vasodilators and can be used for prevention and/or treatment of myocardial infarction.

Piperazine derivatives of theobromine have been disclosed in GB—A—1,289,287, DE—A—1,966,620 and FR—A—2,450,831, essentially as having coronary dilating effects.

SUMMARY OF THE INVENTION

The present invention concerns novel compounds of the formula I

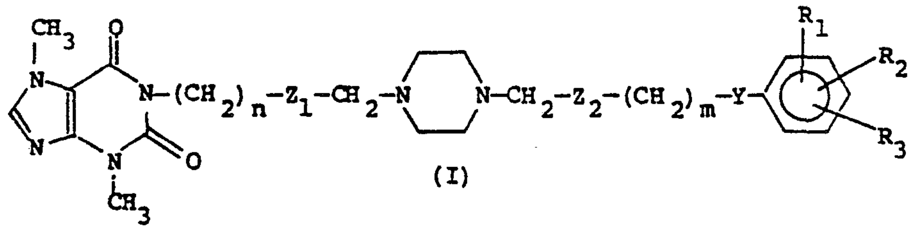

(I)

and the pharmaceutically acceptable acid addition salts thereof, wherein

$Z_1$ and $Z_2$ are each independently selected from the group consisting of $CH_2$, CHOB and C=O, wherein B is selected from the group consisting of hydrogen and alkanoyl;

Y is oxygen or sulfur;

n is an integer from 0—4 but cannot be zero when $Z_1$ is CHOB;

m is an integer from 1—4; and

$R_1$, $R_2$ and $R_3$ are each independently hydrogen, halogen, hydroxy, trifluoromethyl, alkyl or alkoxy.

In another aspect, this invention concerns pharmaceutical compositions containing the above compounds as active ingredients.

In a third aspect, the invention concerns processes for preparing these compounds.

DETAILED DESCRIPTION OF THE INVENTION

Definitions:

As used herein

"Alkyl" means a branched or unbranched saturated hydrocarbon chain containing 1—6 carbon atoms, such as methyl, ethyl, propyl, tert-butyl or n-hexyl.

"Alkoxy" means —OR wherein R is alkyl as herein defined.

"Alkanoyl" means

$$-\overset{O}{\overset{\|}{C}}R$$

wherein R is alkyl as defined herein.

"Halogen" means chloro, bromo or iodo.

"Pharmaceutically acceptable acid addition salt" refers to those salts which retain the biological effectiveness and properties of the free bases, which are not biologically or otherwise undesirable, and which are formed with inorganic acids such as hydrochloric acid, hydrobromic acid, sulfuric acid, nitric acid or phosphoric acid, and organic acids such a acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, menthanesulfonic acid, ethanesulfonic acid, p-toluenesulfonic acid or salicyclic acid.

In the reaction schemes as shown herein below, and in the Claims:

"ThB" represents the theobromic-l-yl moiety:

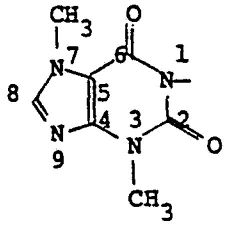

i.e. theobromine, which is linked to the remainder of the molecule through the ring nitrogen as position 1.

"X" represents a halogen atom; i.e. chloro, bromo or iodo; however, each X shown may be selected independently from this group;

"A" represents a moiety selected from the group consisting of

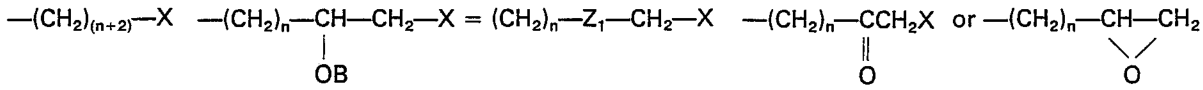

wherein n is as herein defined.

Process for Preparation

Reaction Schemes 1 and 2, shown below, are complementary processes for linking the two "halves" of the compounds of Formula I through the piperazine ring.

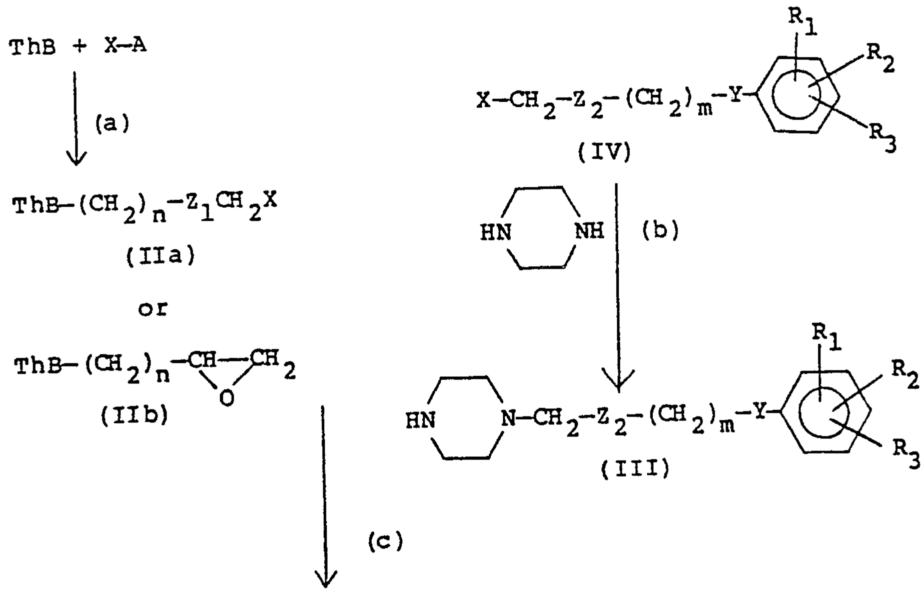


(IIa)

or

(IIb)

(IV)

(III)

(I)

Reaction Scheme 1

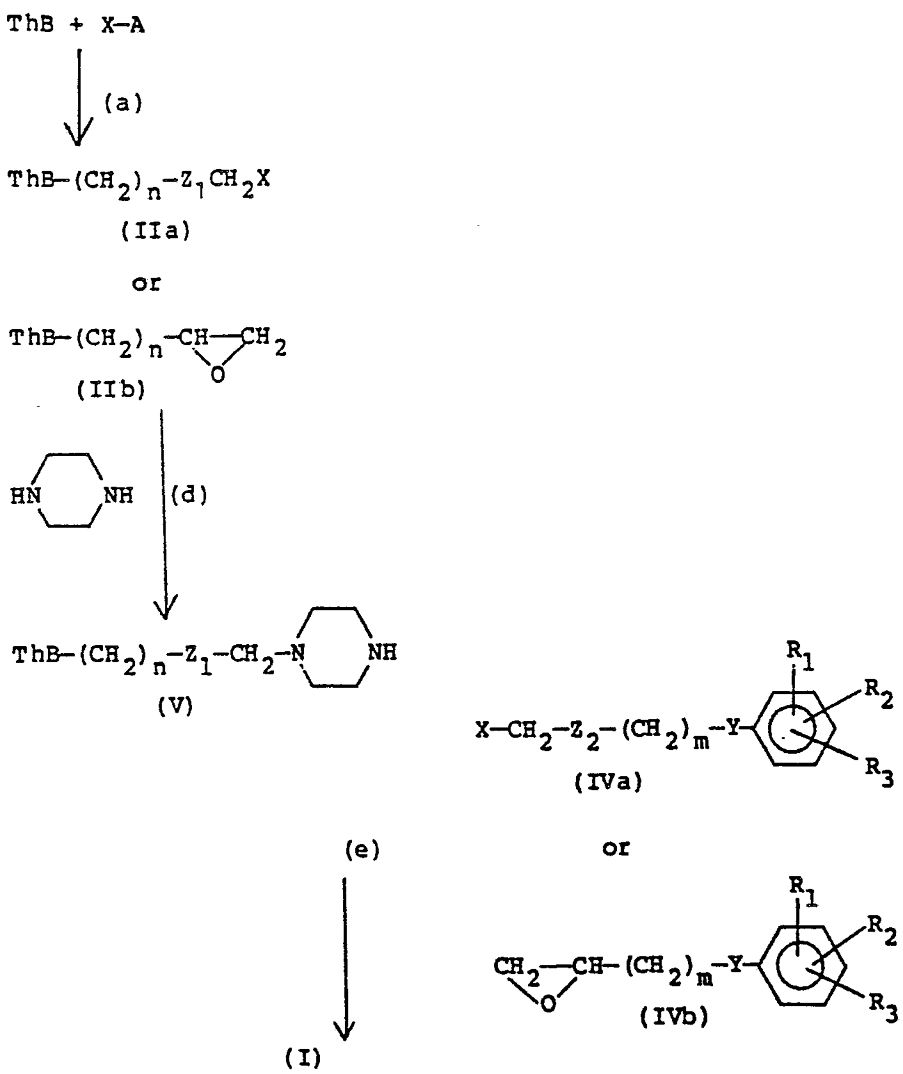


(IIa)

or

(IIb)

(V)

(IVa)

or

(IVb)

(I)

Reaction Scheme 2

In the reaction schemes shown, isolation and purification of the compounds and intermediates described, whether in the body of the specification, or examples, can be effected, if desired, by any suitable separation or purification procedure such as, for example, filtration, extraction, crystallization, column chromatography, thin-layer chromatography or thick-layer chromatography, or a combination of these procedures. Specific illustrations of suitable separation and isolation procedures can be had by reference to the examples hereinbelow. However, other equivalent separation or isolation procedures could, of course, also be used.

The salt products are also isolated by conventional means. For example, the reaction mixtures may be evaporated to a dryness, and the salts can be further purified by conventional methods.

The compounds of the present invention in which $Z_1$ and/or $Z_2$ is CHOB and which, therefore, contain at least one chiral centre, may be prepared in either optically active form or as racemic mixtures. Unless otherwise specified, the compounds described herein are all in the racemic form. However, the scope of the subject invention herein is not to be considered limited to the racemic forms, but to encompass the individual optical isomers of the compounds.

In those embodiments where both $Z_1$ and $Z_2$ are chiral, diastereomeric forms also are possible. The invention herein includes mixtures of diastereomers as well as individual diastereomeric forms. Diastereomers may, of course, be separated by conventional means used to separate compounds.

If desired, the racemic mixtures herein may be resolved into their optical antipodes by conventional resolution means; for example by separation (e.g. fractional crystallization) of the diastereomeric salts formed by the reaction of these compounds with optically active acids. Exemplary of such optically active acids are the optically active forms of camphor-10-sulfonic acid, 2-bromo-camphor-π-sulfonic acid, camphoric acid, menthoxyacetic acid, tartaric acid, malic acid, diacetyltartaric acid or pyrrolidine-5-carboxylic acid. The separated pure diastereomeric salts may then be cleaved by standard means to afford the repective optical isomers of the compounds of Formula I.

Reaction Schemes 1 and 2 have, in common, step (a), the condensation of theobromine with a halide containing the desired side chain. The reaction is carried out in the presence of a polar solvent, such as for example, aqueous alkanol, a pure polar alcohol, polar ketone, or water, preferably aqueous ispropanol, and using a basic catalyst such as, e.g. sodium or potassium hydroxide or carbonate, preferably potassium hydroxide. The reaction is carried out at elevated temperatures of about 70°—120°, most conveniently at the reflux temperature of the solvent. A several-fold molar excess, preferably (2—3 fold) of the halide bearing the side chain, (i.e. the compound of formula X—A), is used.

Compounds of Formula IV are described in European Patent Application 0005385. They are prepared in a manner similar to that described in step (a) for the preparation of compounds of Formula IIa and IIb, but substituting the appropriate phenol or thiophenol for theobromine. As described above, the reaction is carried out in a polar solvent at elevated temperatures with a basic catalyst and, similarly, using a molar excess of the compound of formula X—A over the substrate phenol or thiophenol.

The condensation of the compounds of Formula IV with piperazine to yield compounds of Formula III (step b, reaction scheme 1) and of compounds of Formula II with piperazine to yield compounds of Formula V (step d, reaction scheme 2) are carried out in similar fashion. In each case, an excess of piperazine (about 1.5—4 fold, preferably 2—3 fold molar excess) is heated to reflux with the halide in the presence of a polar solvent such as methyl ethyl ketone (MEK), water or ethanol, preferably alcohol-water. Reaction is continued for about 12—36 hours, preferably 20—25 hours. A basic catalyst, as described above for step (a) is used, in this case, preferably sodium hydroxide. The resulting piperazine adduct is then isolated by conventional means, known to those skilled in the art.

The condensations represented by steps (c) of scheme 1, and step (e) of scheme 2 are again similar, both to each other and to the steps previously described. The reaction conditions approximate those described above as to solvent, catalyst, time and temperature. However, approximately equimolar amounts of the reactants containing the two ends of the molecule are employed.

The reaction schemes, shown offer methods to prepare all of the compounds of the present invention. However, it should be noted, in addition, that compounds of Formula I wherein $Z_1$ and/or $Z_2$ is C=O may be reduced to the corresponding alcohols of Formula I using a metal hydride, such as, for example, $KBH_4$ or $NaBH_4$ in a polar solvent, such as aqueous methanol. The reduction is accomplished by dissolving the substrate carbonyl in the solvent chosen, and adding an excess (the amount of excess depending on the side reaction with solvent) of the hydride in small portions with stirring until reaction is complete. The temperature is kept at about 0°C—25°C, preferably 4°—15°C.

Conversely, compounds of Formula I herein $Z_1$ and/or $Z_2$ is CHOH may be oxidized to the corresponding carbonyls under suitable, mild conditions. Appropriate oxidizing agents include, for example, dilute neutral permanganate or chromic acid, preferably permanganate. The substrate alcohol is dissolved in a polar solvent such as alcohol, MEK, or alkanol-water, and a solution of the oxidizing agent added until reaction is complete. Approximately stoichiometric amounts of oxidizing agent are required. The temperature is kept at about 5°—30° preferably 15°—20°C.

Also, compounds of Formula I wherein $Z_1$ and/or $Z_2$ is CHOH may be esterified to convert them to the alkanoyl derivatives. This is accomplished by heating the compound of Formula I with a molar excess of the appropriate carboxylic anhydride or chloride in a tertiary amine solvent, such as, for example, pyridine. The temperature is kept at about 20°—90°, preferably 15°—30°.

Conversely, the compounds of Formula I wherein $Z_1$ and/or $Z_2$ is

$$CHO-\overset{O}{\overset{\|}{C}}R$$

may be hydrolyzed, using conventional methods, well known to those in the art to the corresponding alcohols: The ester is heated in a water solution with an acid or basic catalyst until hydrolysis is complete.

Salts of the compounds of Formula I are prepared by reacting the corresponding free bases with appropriate acids or acid salts at a temperature of between 0° and 100°C. Conversely, free bases can be prepared by reacting corresponding acid addition salts with suitable alkaline agents, such as sodium or potassium hydroxide at 0°—100°C.

The products of Formula I, synthesized by any of the pathways disclosed herein are optionally converted, when appropriate, to the free base, or to any salt, said salts including, but not being limited to the pharmaceutically acceptable acid addition salts.

Preferred Embodiments

Preferred embodiments of the compounds of the invention are those wherein n and m are 1, and $Z_1$ and $Z_2$ are CHOH or $CH_2$, and the pharmaceutically acceptable acid addition salts thereof.

Especially preferred are the following and their pharmaceutically acceptable acid addition salts:

1-(3-theobromin-1-yl-2-hydroxypropyl)-4-(3-phenylthiopropyl)piperazine.

Utility and Administration

The compounds of the present invention are particularly effective antihistamines. They have been demonstrated to antagonize the effects of histamine in a variety of tests related to such activity, including their activity in prevention of anaphylactic shock in rats, bronchodilation in guinea pigs, inhibition of muscle contraction in response to stress in rats, and brachycardial effects in guinea pigs. Therefore, the compounds are useful in the treatment of respiratory diseases and allergic reactions in mammals, including, but not limited to, asthma, hay fever, and the common cold.

Administration of the active compounds and salts described herein can be via any of the accepted modes of administration for antihistaminic agents which relieve congestion or otherwise effect the control of allergic or other respiratory symptoms. These methods include oral, parenteral and otherwise systemic, or aerosol forms. Depending on the intended mode, the compositions may be in the form of solid, semisolid or liquid dosage forms, such as, for example, tablets, suppositories, pills, capsules, powders, liquids or suspensions, preferably in unit dosage forms suitable for single administration of precise dosages. The compositions will include a conventional pharmaceutical carrier or excipient and an active compound of Formula I or the pharmaceutically acceptable salts thereof and, in addition, may include other medicinal agents.

The amount of active compound administered will of course, be dependent on the subject being treated, the severity of the affliction, the manner of administration and the judgment of the prescribing physician. However, an effective dosage is in the range of 0.1—500 µg/kg/day, preferably 5—100 µg/kg/day. For an average 70 kg human, this would amount to 7 µg to 35 mg per day, or preferably 35 µg to 7 mg/day.

Typical compositions contain 0.01—95% by weight of active ingredient, with the balance one or more acceptable non-toxic carriers. The percentage of active ingredient, will, of course, depend upon the dosage form and the mode of administration.

For solid compositions, conventional non-toxic solid carriers include, for example, pharmaceutical grades of mannitol, lactose, starch, magnesium stearate, sodium saccharin, talcum, cellulose, glucose, sucrose or magnesium carbonate may be used. The active compound as defined above may be formulated as suppositories using, for examples, polyalkylene glycols, for example, propylene glycol, as the carrier. Liquid pharmaceutically administerable compositions can, for example, be prepared by dissolving, dispersing, etc. an active compound as defined above and optional pharmaceutical adjuvants in a carrier such as, for example, water, saline, aqueous dextrose or glycerol, ethanol, to thereby form a solution or suspension. If desired, the pharmaceutical composition to be administered may also contain minor amounts of nontoxic auxiliary substances such as wetting or emulsifying agents or pH buffering agents, for example, sodium acetate, sorbitan monolaurate, triethanolamine sodium acetate, sorbitan monolaurate triethanolamine oleate. Actual methods of preparing such dosage forms are known, or will be apparent, to those skilled in this art; for example, see *Remington's Pharmaceutical Sciences,* Mack Publishing Company, Easton, Pennsylvania, 15th Edition, 1975. The composition or formulation to be administered will, in any event, contain a quantity of the active compound(s) in an amount effective to alleviate the symptoms of the subject being treated.

For oral administration, a pharmaceutically acceptable non-toxic composition is formed by the incorporation of any of the normally employed excipients, such as, for example pharmaceutical grades of mannitol, lactose, starch, agnesium stearate, sodium saccharin, talcum, cellulose, glucose, sucrose or magnesium carbonate. Such compositions take the form of solutions, suspensions, tablets, pills, capsules,

powders or sustained release formulations. Such compositions may contain 10%—95% active ingredient, preferably 1—70%.

Parenteral administration is generally characterized by injection, either subcutaneously intramuscularly or intravenously. Injectables can be prepared in conventional forms, either as liquid solutions or suspensions, solid forms suitable for solution or suspension in liquid prior to injection, or as emulsions. Suitable excipients are, for example, water, saline, dextrose, glycerol or ethanol. In addition, if desired, the pharmaceutical compositions to be administered may also contain minor amounts of non-toxic auxiliary substances such as wetting or emulsifying agents or pH buffering agents, such as for example, sodium acetate, sorbitan monolaurate or triethanolamine oleate.

A more recently devised approach for parenteral administration employs the implantation of a slow-release or sustained-release system, such that a constant level of dosage is maintained. See, e.g., U.S. Patent No. 3,710,795.

For systemic administration via suppository, traditional binders and carriers include, e.g. polyalkalene glycols or triglycerides. Such suppositories may be formed from mixtures containing active ingredient in the range of 0.5%—10%; preferably 1—2%.

For aerosol administration, the active ingredient is preferably supplied in finely divided form along with a surfactant and a propellant. Typical percentages of active ingredients are 0.01 to 20% by weight, preferably 0.04 to 1.0%.

Surfactants must, of course, be non-toxic, and preferably soluble in the propellant. Representative of such agents are the esters or partial esters of fatty acids containing from 6 to 22 carbon atoms, such as aproic, octanoic, lauric, palmitic, stearic, linoleic, linolenic, olestearic and oleic acids with an aliphatic polyhydric alcohol or its cyclic anhydride such as, for example, ethylene glycol, glycerol, erythritol, arabitol, mannitol, sorbitol, the hexitol anhydrides derived from sorbitol (the sorbitan esters sold under the trademark "Spans") and the polyoxyethylene and polyoxypropylene derivatives of these esters. Mixed esters, such as mixed or natural glycerides may be employed. The preferred surface-active agents are the oleates of sorbitan, e.g., those sold under the trademarks "Arlacel C" (Sorbitan sesquiolate), "Span 80" (sorbitan monooleate) and "Span 85" (sorbitan trioleate). The surfactant may constitute 0.1—20% by weight of the composition, preferably 0.25—5%.

The balance of the composition is ordinarily propellant. Liquefied propellants are typically gases at ambient conditions, and are condensed under pressure. Among suitable liquefied propellants are the lower alkanes containing up to five carbons, such as butane and propane; and preferably fluorinated or fluorochlorinated alkanes, such as are sold under the trademark "Freon." Mixtures of the above may also be employed.

In producing the aerosol, a container equipped with a suitable valve is filled with the appropriate propellant, containing the finely divided active ingredient and surfactant. The ingredients are thus maintained at an elevated pressure until released by action of the valve.

For use in the prevention and/or treatment of myocardial infarction, oral administration is the preferred mode. The oral compositions and excipients contained therein are similar to those decribed hereinabove. The dosage level is, again dependent on the specific circumstances which pertain, but would be in the range of 0.0001 to 50 mg/kg per day.

## EXAMPLES

The following Examples are illustrative of the invention, but are not to be construed as limiting it:

### Preparation A

Preparation of 1-(3-Chloropropyl)-Theobromine (Step a, Preparation of IIa)

A well stirred reaction medium containing 1 mole theobromine, 3 moles 1-bromo-3-chloro-propane, 600 ml isopropyl alcohol and 60 ml water is refluxed for 24 hours.

An aqueous solution of potassium hydroxide (1.2 mole) is then slowly added dropwise thereto.

The alcohol solvent is then removed, the resulting material is extracted with methylene chloride, washed with water, after which the solvent is evaporated off, and the product is recrystallized from methanol.

### Preparation B

Preparation of 1-(3-Phenylthiopropyl)-Piperazine (Step b, Preparation of III)

To a solution containing 1.1 mole sodium hydroxide in 500 ml water are added 1 mole thiophenol and 2 moles 1-bromo-3-chloro-propane. The mixture is then refluxed for 30 hours, with vigorous stirring. After cooling, the resulting material is extracted with methylene chloride. After washing with silute lye, and then with water, the solvent is evaporated *in vacuo.* The chlorinated derivative distills at 138—140°C under 13 mm Hg.

To 1 liter 50% aqueous alcohol are added 3 moles piperazine, 1 mole 3-phenylthio-1-chloropropane, 1 mole 10N sodium hydroxide. The mixture is refluxed for 24 hours, with stirring. The ethanol is then evaporated off, and the resulting material is exacted with methylene chloride. The organic phase is thoroughly washed with water, and is then concentrate and distilled: bp 0.05 mm = 140—142°C.

Preparation C

Preparation of (3-Theobromin-1-yl-2-Hydroxypropy)Chloride (Step a, Preparation of IIb)

0.20 moles of theobromine is dissolvedin 600 ml ethanol, which contains 0.20 moles of sodium hydroxide by stirring at 50°C. The solvent is evaporated and the residue dried.

The above residue is refluxed in 700 ml of epichloro hydrin. The NaCl precipitate is filtered off and the filtrate evaporated to give a residue, which is recrystallized from ethanol to give the title compound.

Example 1

Preparation of 1-(3-theobromine-1-yl-2-hydroxypropyl)-4-(3-phenylthiopropyl)piperazine (Step c, Reaction Scheme I)

(3-theobromin-1-yl-2-hydroxypropyl)chloride (1 mole) and 1-(3-phenylthiopropyl)-piperazine are refluxed for 24 hours in a 50% aqueous-alcoholic solution. On completion of the reaction, sodium hydroxide (1 mole) is then added. The mixture is extracted with methylene chloride and washed with water, after which the solvent is evaporated off and the residue is recrystallized from ethanol.

The dihydrochloride is prepared in the manner described in Example 3, m.p. = 226°C.

Example 2

7-[3-]4-(3-phenylthiopropyl)-1-piperazinyl-2-hydroxypropyl-theobromine (Step e, Reaction Scheme 2)

A solution of ethanol (2 litres) containing 1-(3-theobromin-1-yl-2-hydroxypropyl)piperazine (1 mole) and 3-(phenylthio)propylchloride (1 mole is refluxed for 5 hours. The ethanol is partly removed, and crystallization takes place. The resulting crystals are suction filtered and may be recrystallized from ethanol.

The dihydrochloride is prepared in the manner described in Example 3, m.p. = 234°C.

Example 3

Conversion of Free Base to Salt.

Excess 3% hydrogen chloride in methanol is added to a solution of 1.0 g 1-(3-theobromin-1-yl-2-hydroxypropyl)-4-(3-phenylthiopropyl)piperazine in 20 ml methanol. Diethyl ether is added until precipitation is complete. The product dihydrochloride is filtered, washed with ether, air dried and recrystallized, m.p. 226°C.

In a similar manner, all compounds of Formula I in free base form may be converted to the acid addition salts by treatment with the appropriate acid, sulfuric acid, nitric acid, phosphoric acid, acetic acid, propionic acid, glycolic acid, pyruvic acid, oxalic acid, malonic acid, succinic acid, malic acid, maleic acid, fumaric acid, tartaric acid, citric acid, benzoic acid, cinnamic acid, mandelic acid methanesulfonic acid, ethanesulfonic acid or p-toluenesulfonic acid.

Example 4

Conversion of Salt to Free Acid.

1.0 g or 1-(3-theobromine-1-yl-2-hydroxypropyl)-4-(3-phenylthiopropyl)piperazine 2HCl suspended in 50 ml ether is stirred with excess dilute aqueous potassium carbonate solution until the salt is completely dissolved. The organic layer is then separated, washed twice with water, dried over magnesium sulfate and evaporated to yield 1-(3-theobromin-1-yl-2-hydroxypropyl)-4-(3-phenylthiopropyl)piperazine as the free base.

Example 5

Conversion of Alcohol to Ester.

A) 1.0 grams of 1-(3-theobromin-1-yl-2-hydroxypropyl)-4-(3-phenylthiopropyl-piperazine is dissolved in 30 ml pyridine. 2 ml of acetic anhydric is then added. The mixture is kept at room temperature for 20 hours. Solvent is evaporated and the ester 1-(3-theobromine-1-yl-2-acetoxypropyl)-4-(-3-phenylthiopropylyl)piperazine, is then isolated, recrystallized by conventional techniques, as the dihydrochloride.

B) In a manner similar to that described in part A of this Example, the orresponding n-propionyloxy; i-butyryloxy; n-oxy,and n-caproyloxy valeryl compounds are derived from 1-(3-theobromin-1-yl-2-hydroxypropyl)-4-(3-phenylthiopropyl)piperazine are prepared.

Example 6

Other compounds of Formula I may also be prepared according to the procedures of Examples 1 to 5. Exemplary of these are the following compounds for which m = n = 1;

| No. | $Z_1$ | Y | $Z_2$ | $R_1$ | $R_2$ | $R_3$ | Nature of the salt | M.P. |
|---|---|---|---|---|---|---|---|---|
| 1 | $CH_2$ | S | $CH_2$ | H | H | H | 2HC1 | 234°C |
| 2 | CHOH | O | $CH_2$ | H | H | H | 2HCl | 234°C |
| 3 | CHOH | S | $CH_2$ | H | H | H | 2CHI | 226°C |
| 4 | $CH_2$ | O | $CH_2$ | H | H | H | 2CHI | 224°C |
| 5 | CHOH | S | $CH_2$ | 4-Cl | H | H | 2HCl | 240°C |
| 6 | CHOH | O | $CH_2$ | 4-$OCH_3$ | H | H | 2HCl | 250°C |
| 7 | CHOH | O | $CH_2$ | 4-Cl | H | H | 2HCl | 248°C |
| 8 | CHOH | O | $CH_2$ | 4CL | 3-$CH_3$ | 5-CH3 | 2HCl | 250°C |
| 9 | CHOH | S | $CH_2$ | 4-CH-3 | H | H | 2HCl | 240°C |
| 10 | $CH_2$ | O | $CH_2$ | 4-CL | H | H | 2HCl | 250°C |
| 11 | $CH_2$ | S | $CH_2$ | 4-CL | H | H | 2HCl | 235°C |
| 12 | $CH_2$ | S | $CH_2$ | 4-$CH_3$ | H | H | 2HCl | 230°C |
| 13 | $CH_2$ | S | $CH_2$ | H | H | H | 2HCl | 245°C |

Example 7

Pharmaceutical Compositions

The active ingredient in this example is 1-(3-theobromin-1-yl-2-hydroxypropyl)-4-(3-phenylthiopropyl)piperazine. The other compounds of this invention may, of course, also be used.

A. Capsules

| | |
|---|---|
| Active Ingredient | 30.0 mg |
| Lactose, special | 163.0 mg |
| Talc | 5.0 mg |
| Magnesium stearate | 2.0 mg |

B. Injectable ampoules

| | |
|---|---|
| Active Ingredient | 10.0 mg |
| Sodium chloride | 35.0 mg |
| Monosodium phosphate, to pH 5.5—6 | |
| Distilled water, qs ad | 5.0 ml |

C. Tablets

| | |
|---|---|
| Active Ingredient | 10.0 mg |
| Lactose | 80.0 mg |
| Cellulose | 97.5 mg |
| Silica | 1.5 mg |
| Starch | 10.0 mg |
| Magnesium stearate | 1.0 mg |

D. Drinkable Suspension

| | |
|---|---|
| Active Ingredient | 200.0 mg |
| Benzoic acid | 250.0 mg |
| Polyoxyethylene glycol and water, qs ad | 200.0 ml |

E. Aerosol I

| | |
|---|---|
| Active Ingredient | 0.6% |
| Span 85 | 0.5% |
| Freon 11 | 20.0% |
| Freon 12/Freon 114 (20/80) | 78.9% |

Aerosol II

| | |
|---|---|
| Active Ingredient | 0.88% |
| Sodium sulfate (anhydrous), micronized | 0.88% |
| Span 85 | 1.00% |
| Propellant consisting of 50% Freon 12, 25% Freon 11, and 25% Freon 114 | 97.24% |

Aersol III

| | |
|---|---|
| Active Ingredient | 0.50% |
| Span 80 | 0.50% |
| Propellant (C) consisting of 30% Freon 11 and 70% Freon W | 99.0% |

Aerosol IV

| | |
|---|---|
| Active Ingredient | 3.0% |
| Span 85 (sorbitan trioleate) | 1.0% |
| Fron 11 (trichloromonofluoromethan) | 30.0% |
| Freon 114 (dichlorotetrafluoroethane) | 41.0% |
| Freon 12 (dichlorodifluoromethane) | 25.0% |

Example 8

Bronchodilator action

The method of Konzett and Rossler is used for the test. Tri-coloured male guinear pigs weighing 250—300 g (C.E.R.J. Janvier) with ethyl carmate (1—2 g/kg/i.p.). A tracheal cannula is positioned and connected to a respiratory pump for small animals (Ideal — Palmer type).

The animals are maintained under forced respiration, at a determined rate and frequency level.

An electromagnetic cell is mounted in line on the blowing circuit; the signal released by this sensor is amplified with a pressure preamplifier and amplifier before being received by a potentiometric recorder.

The bronchoconstrictor agents are administered intravenously (jugular vein) after the animals have

been allowed to rest for a certain period of time and a gallamine injection (1 mg × $kg^{-1}$/i.v.) in order to prevent, by curarization, the specifically involuntary muscular reactions. After a constant bronchospasm has been obtained, the test material is iteratively administered by the intravenous (or intragastric) route until the reference bronchospasm is again obtained. The spasm-inducing agents are histamine (5 µg × $kg^{-1}$/i.v.), serotonine (1.0 µg × $kg^{-1}$/i.v.) and acetyl choline (20 µg × $kg^{-1}$/i.v.).

The percent inhibition of the reference bronchospasm is a measure of the activity of the test material.

The compounds of the Formula I and more particularly compounds 3 and 4 of Example 6, at single doses of 0.0125—0.250 mg/kg/iv., provide a protection against bronchoconstrictor agents such as histamine, acetyl choline and serotonine.

Example 9

Antianaphylactic action

The test is effected with male Sprague Dawley rats (IFFA CREDO) weight 250 g. At time 0, the animals are sensitized with an injection of egg-albumin (1 mg, s.c.) and of aluminum hydroxide (200 mg, s.c.), simultaneously with an injection of *Haemophilus pertussis* vaccine (Vaxicoq® PASTEUR 1.5 × $10^{10}$ organisms/0.5 ml, at a rate of 1.5 ml/i.p./rat). After a period of time of days, the rats are anesthetized with ether and a blood sample is obtained by puncturing the descending aorta.

After centrifugation and dilution, the antiserum is reinjected to other male Sprague Dawley rats (180—200 g) by the intradermal route (0.1 ml/i.d.) and on 3 different sites of the dorsal area. After 24 hours, the test material is administered orally, 30 minutes prior to an intradermal histamine injection on three other sites of the dorsal area (30 µg/kg/i.d.) and a simultaneous intravenous injection of a physiological solution containing 5 mg egg-albumin and 2.5 mg Evans Blue (1 ml/rat); after a period of time of 30 minutes, the animals are sacrificed by administration of an overdose of ether; the skin of the back is cut off, turned inside out and then spread out.

Under the influence of the antigen (egg-albumin), the 3 sites corresponding to the prior antiserum (anti-egg-albumin) exhibit a blue color due to the local diffusion of the Evans Blue (histamine release by the subcutaneous mastocytes). The 3 other sites corresponding to the local action of histamine, show the good reactivity of the controls together with the specific antihistaminic action of the test material.

The surface area of each spot is calculated according to the formula:

$$L \times 1 \times \pi/4$$

in which *L* and *1* represent the long and the short axis, respectively, of the ellipse. The best dilution of the reference antiserum corresponding to a surface area of about 100 $mm^2$. The colour strength is in turn evaluated according to the following scale.

| | | |
|---|---|---|
| 0 | : | nil |
| 0.5 | : | very pale blue |
| 1 | : | pale blue |
| 1.5 | : | deep blue |
| 2 | : | very deep blue |

The antianaphylactic action of the product is obtain by averaging the percent inhibition obtained on the surface of the spots and their color strength.

The compounds of the Formula I, and more particularly compounds 3 and 4 of Example 6, at single dose of 30 mg/kg, induce an inhibition.

Example 10

Musculoptropic action

The investigation is conducted on the isolated duodenum of rat, with respect to barium chloride-induced contractions.

Male Sprague Dawley rats, weighing 300—400 g and which have been kept fasting for 24 hours are used to test animals.

After decapitation of the animal, a portion of the duodenum (3—4 cm long) is taken and positioned in a test tube for isolated organ containing Tyrode liquid, thermostatically maintained at a temperature of 36.5°C under an air atmosphere. The other end of the duodenum is connected to an isometric stress gauge; at the start, the organ is stretched up to 500 mg. The $BaCl_2$ dosage (about 200 µg × $ml^{-1}$ of bath) is selected in such a manner as to obtain a constant response.

The test material, dissolved in Tyrode liquid, is added at increasing dosages, preventively to the contractions of the organ. The effect of the products is given as percent inhibition of the reference contraction.

Compounds 3 and 4 in Example 6 produce 50% relaxation at doses of 110 µg/ml and 21 µg/ml respectively.

Example 11

Chronotropic action

The investigation is effected on the isolated auricle of guinea pigs. The animals weight 300—500 g and are sacrificed by decapitation. The right auricle is taken and placed in Tyrode's liquid, under an atmosphere containing 95% $O_2$ and 5% $CO_2$ and thermostatically maintained at 30°C. The auricle is attached to the bottom of the isolated organ cell, and the other end is connected to an isometric gauge.

The auricle beats in a spontaneous manner (automatically of the pace-maker cells of the nodal tissue) and the cardiac frequency is recorded from the signals released by the sensor, amplified and integrated *via* a cardio-tachymeter. The test materials, dissolved in Tyrode's liquid, are generally administered cumulatively until a maximum effect is produced.

The compounds of the Formula I, and more particularly compounds 1, 3 and 4 of Example 6 have a negative chronotropic effect (bradycardia).

Example 12

Toxicity

Male Sprague Dawley rats (IFFA CREDO France) weighing about 150 g are used as test animals. The animals are fasted the day prior to the test.

On the day of the treatment, they are orally administered a single dose of product suspended in gum water containing 3% gum arabic. The animals are then observed in individual cages to detect the disorders induced by the toxic action of the product; the deaths which may occur are recorded for a period of time of 7 days.

At an oral dose of 750 mg/kg, compounds 2, 3 and 4 in Example 6 do not cause any deaths.

Example 13

Vasodilator Activity

The rabbit's ear is perfused continuously with a Krebs solution containing norepinephrine and an increase of the perfusion pressure is induced by the vasoconstrictor effect of norepinephrine. The compounds are injected at different dose levels after the injection of piribedil as control.

A decrease of the perfusion pressure is induced by the vasodilator effect of the compounds tested. Compounds 5, 7 and 8 of Example 6 show the same activity as Piribedil and compound 9 of Example 6 show a better activity than Piribedil.

Example 14

Smooth Muscle Antagonism

The neurotropic effect of the compounds is tested in vitro in isolated duodenum of rats and atropine is used as reference. The antihistamine effect is tested on isolated ileum of guinea pigs and promethazine is used as reference.

Compounds 5, 6, 7, 8, 9, 10, 11 and 12 of Example 6 all show some neurotropic and antihistamine effects.

**Claims for the Contracting States: BE CH DE FR GB IT LI LU NL SE**

1. A compound of the formula I

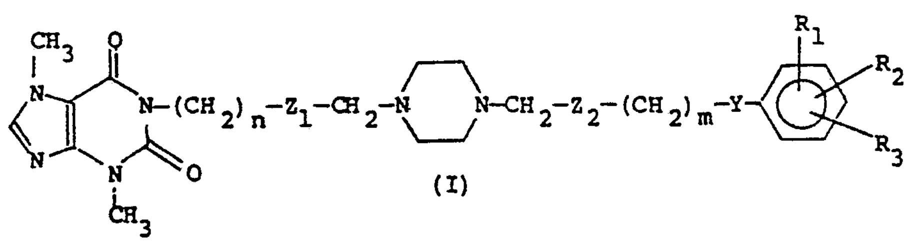

(I)

and the pharmaceutically acceptable acid addition salts thereof, wherein

$Z_1$ and $Z_2$ are each independently selected from the group consisting of $CH_2$, CHOB and C=O, wherein B is selected from the group consisting of hydrogen and

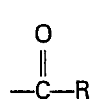

wherein R is $C_{1-6}$ alkyl;

Y is oxygen or sulfur;

n is an integer from 0—4 but cannot be zero when $Z_1$ is CHOB;

m is an integer from 1—4; and

$R_1$, $R_2$ and $R_3$ are each independently hydrogen, halogen, hydroxy, trifluoromethyl, $C_{1-6}$ alkyl or $C_{1-6}$ alkoxy.

2. The compound of Claim 1 and the pharmaceutically acceptable acid addition salts thereof wherein $Z_1$ and $Z_2$ are each independently $CH_2$ or CHOH; and m and n are both 1.

3. The compound of Claim 2 and the pharmaceutically acceptable acid addition salts thereof which is 1-(3-theobromin-1-yl-2-hydroxypropyl)-4-(3-phenylthiopropyl) piperazine.

4. A pharmaceutical composition which comprises an effective amount of the compound of claim 1 or a pharmaceutically acceptable acid addition salt thereof, in admixture with at least one pharmaceutically acceptable, non-toxic excipient.

5. A process for preparing a compound as claimed in claim 1, which process comprises

(a) reacting a compound of the formula IIa

$$ThB—(CH_2)_n—Z_1—CH_2X \qquad (IIa)$$

or IIb

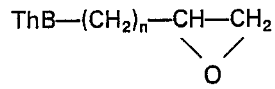 (IIb)

wherein $Z_1$ and n are as herein defined with a compound of the formula III

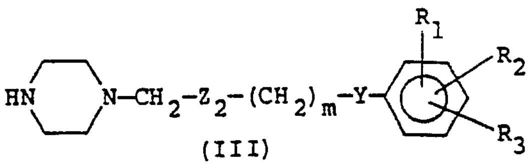

(III)

wherein $Z_2$, m and Y are as herein defined; or

(b) reacting a compound of the formula V

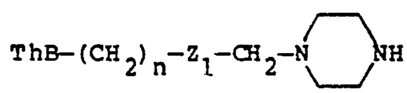

(V)

wherein $Z_1$ and n are as herein defined with a compound of the formula IVa

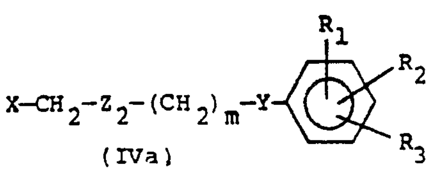

(IVa)

or IV b

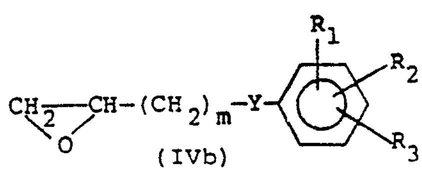

(IVb)

wherein Z, m and Y are as herein defined; or

(c) oxidizing a compound of Formula I wherein $Z_1$ and/or $Z_2$ is CHOH and Y, n, m, $R_1$, $R_2$ and $R_3$ have the above meanings to a compound of Formula I wherein $Z_1$ and/or $Z_2$ is C=O and Y, n, m, $R_1$, $R_2$ and $R_3$ have the above meanings; or;

(d) reducing a compound of Formula I wherein $Z_1$ and/or $Z_2$ is C=O and Y, n, m, $R_1$, $R_2$ and $R_3$ have the above meanings to a compound of Formula I wherein $Z_1$ and/or $Z_2$ is CHOH and Y, n, m, $R_1$, $R_2$ and $R_3$ have the above meanings; or

(e) esterifying a compound of Formula I wherein $Z_1$ and/or $Z_2$ is CHOH and Y, n, m, $R_1$, $R_2$ and $R_3$ have the above meanings to a compound of Formula I wherein $Z_1$ and/or $Z_2$ is

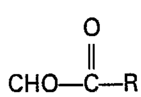

and Y, n, m, $R_1$, $R_2$ and $R_3$ have the above meanings; or

(f) hydrolyzing a compound of Formula I wherein $Z_1$ and/or $Z_2$ is 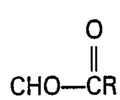

and Y, n, m, $R_1$, $R_2$ and $R_3$ have the above meanings to a compound of Formula I wherein $Z_1$ and/or $Z_2$ is CHOH and Y, n, m, $R_1$, $R_2$ and $R_3$ have the above meanings; or

(g) converting the free base of the compound of Formula I to a pharmaceutically acceptable acid addition salt; or

(h) converting a salt of the compound of Formula I to a free base; or

(i) converting a salt of the compound of Formula I to another salt.

6. A process for preparing a pharmaceutical composition according to claim 4, wherein the active ingredient prepared in accordance with claim 5 is mixed with a pharmaceutically acceptable carrier.

7. The use of a compound of claim 1, 2 or 3 in the preparation of a pharmaceutical composition.

**Claims for the Contracting State: AT**

1. A process for preparing a compound of the formula I

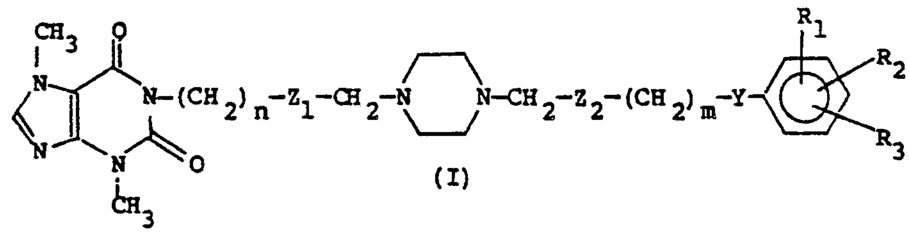

and the pharmaceutically acceptable acid addition salts thereof, wherein $Z_1$ and $Z_2$ are each independently selected from the group consisting of $CH_2$, CHOB and C=O, wherein B is selected from the group consisting of hydrogen and

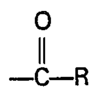

wherein R is $C_{1-6}$ alkyl;

Y is oxygen or sulfur;

n is an integer from 0—4

m is an integer from 1—4 but cannot be zero when $Z_2$ is CHOB; and

$R_1$, $R_2$ and $R_3$ are each independently hydrogen, halogen, hydroxy, trifluoromethyl, $C_{1-6}$ alkyl or $C_{1-6}$ alkoxy, which process comprises

(a) reacting a compound of the formula IIa

$$ThB—(CH_2)_n—Z_1—CH_2X \qquad (IIa)$$

or IIb

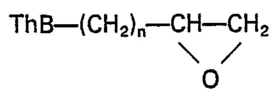

(IIb)

wherein $Z_1$ and n are as herein defined with a compound of the formula III

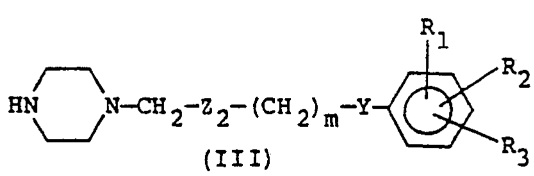

wherein $Z_2$, m and Y are as herein defined; or

(b) reacting a compound of the formula V

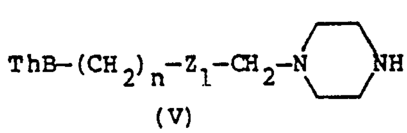

wherein $Z_1$ and n are as herein defined with a compound of the formula IVa

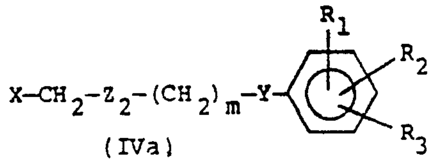
(IVa)
or IV b
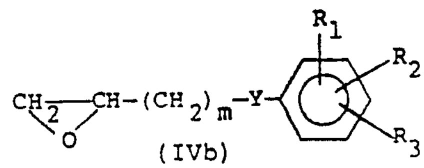
(IVb)

wherein Z, m and Y are as herein defined; or

(c) oxidizing a compound of Formula I wherein $Z_1$ and/or $Z_2$ is CHOH and Y, n, m, $R_1$, $R_2$ and $R_3$ have the above meanings to a compound of Formula I wherein $Z_1$ and/or $Z_2$ is C=O and Y, n, m, $R_1$, $R_2$ and $R_3$ have the above meanings; or;

(d) reducing a compound of Formula I wherein $Z_1$ and/or $Z_2$ is C=O and Y, n, m, $R_1$, $R_2$ and $R_3$ have the above meanings to a compound of Formula I wherein $Z_1$ and/or $Z_2$ is CHOH and Y, n, m, $R_1$, $R_2$ and $R_3$ have the above meanings; or

(e) esterifying a compound of Formula I wherein $Z_1$ and/or $Z_2$ is CHOH and Y, n, m, $R_1$, $R_2$ and $R_3$ have the above meanings to a compound of Formula I wherein $Z_1$ and/or $Z_2$ is

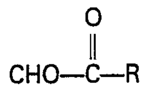

and Y, n, m, $R_1$, $R_2$ and $R_3$ have the above meanings; or

(f) hydrolyzing a compound of Formula I wherein $Z_1$ and/or $Z_2$ is 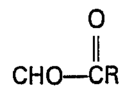

and Y, n, m, $R_1$, $R_2$ and $R_3$ have the above meanings to a compound of Formula I wherein $Z_1$ and/or $Z_2$ is CHOH and Y, n, m, $R_1$, $R_2$ and $R_3$ have the above meanings; or

(g) converting the free base of the compound of Formula I to a pharmaceutically acceptable acid addition salt; or

(h) converting a salt of the compound of Formula I to a free base; or

(i) converting a salt of the compound of Formula I to another salt.

2. A process according to claim 1 for preparing a compound of the Formula I, wherein $Z_1$ and $Z_2$ are each independently $CH_2$ or CHOH and m and n are both 1.

3. A process according to claim 1 for preparing a compound of the Formula I, which is 1-(3-theobromin-1-yl-2-hydroxypropyl)-4-(3-phenylthiopropyl)-piperazine.

4. A process for preparing a pharmaceutical composition, which comprises admixing an effective amount of a compound as defined in claim 1 with at least one pharmaceutically acceptable carrier.

5. The use of a compound as defined in claim 1 in the preparation of a pharmaceutical composition.

**Patentansprüche für die Vertragsstaaten: BE CH DE FR GB IT LI LU NL SE**

1. Verbindung der Formel I

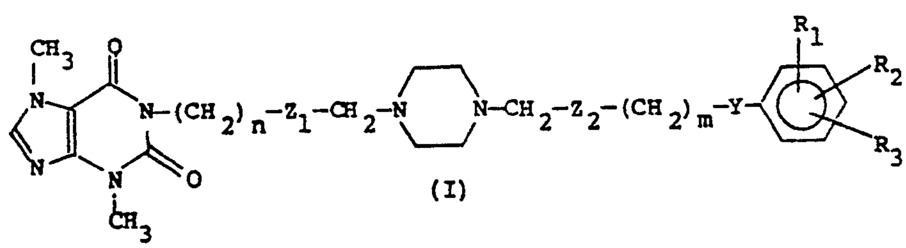
(I)

und die pharmazeutisch annehmbaren Säureadditionssalze derselben, in welcher

$Z_1$ und $Z_2$ jeweils unabhängig voneinander ausgewählt sind aus der Gruppe bestehend aus $CH_2$, CHOB und C=O, wobei B ausgewählt ist aus der Gruppe bestehend aus Wasserstoff und

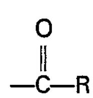

wobei R $C_{1-6}$ Alkyl bedeutet;

Y für Sauerstoff oder Schwefel steht;
n eine ganze Zahl von 0 bis 4 ist, aber nicht gleich 0 sein kann, wenn $Z_1$ gleich CHOB ist;
m eine ganze Zahl von 1 bis 4 ist; und
$R_1$, $R_2$ und $R_3$ jeweils unabhängig voneinander Wasserstoff, Halogen, Hydroxy, Trifluormethyl, $C_{1-6}$ Alkyl oder $C_{1-6}$ Alkoxy sind.

2. Verbindung nach Anspruch 1 und die pharmazeutisch annehmbaren Säureadditionssalze derselben, in welcher $Z_1$ und $Z_2$ jeweils unabhängig voneinander $CH_2$ oder CHOH sind; und m und n beide gleich 1 sind.

3. Verbindung nach Anspruch 2 und die pharmazeutisch annehmbaren Säureadditionssalze derselben, nämlich 1-(3-Theobromin-1-yl-2-hydroxypropyl)-4-(3-phenylthiopropyl)piperazin.

4. Pharmazeutische Zusammensetzung, welche umfaßt eine wirksame Menge einer Verbindung nach Anspruch 1 oder eines pharmazeutisch annehmbaren Säureadditionssalzes derselben, in Mischung mit wenigstens einem pharmazeutisch annehmbaren, nicht-toxischen Exzipienten.

5. Verfahren zur Herstellung einer Verbindung nach Anspruch 1, welches Verfahren umfaßt:
a) die Umsetzung einer Verbindung der Formel IIa

$$ThB—(CH_2)_n—Z_1—CH_2X \qquad (IIa)$$

oder IIb

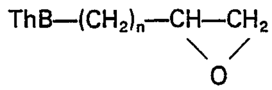

(IIb)

in welchen $Z_1$ und n wie oben definiert sind, mit einer Verbindung der Formel III

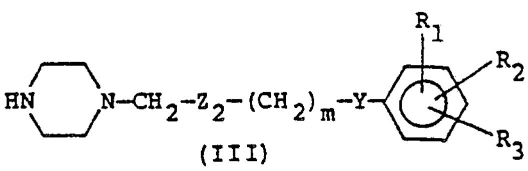

(III)

in welcher $Z_2$, m und Y wie oben definiet sind; oder
b) die Umsetzung einer Verbindung der Formel V

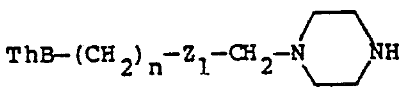

(V)

in welcher $Z_1$ und n wie oben definiert sind, mit einer Verbindung der Formel IVa

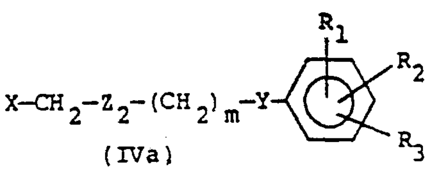

(IVa)

oder IVb

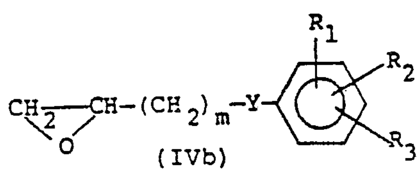

(IVb)

in welchen Z, m und Y wie oben definiert sind; oder
c) die Oxidation einer Verbindung der Formel I, in welcher $Z_1$ und/oder $Z_2$ CHOH sind und Y, n, m, $R_1$, $R_2$ und $R_3$ die obigen Bedeutungen haben, zu einer Verbindung der Formel I, in welcher $Z_1$ und/oder $Z_2$ C=O bedeuten und Y, n, m, $R_1$, $R_2$ und $R_3$ die obigen Bedeutungen haben; oder
d) die Reduktion einer Verbindung der Formel I, in welcher $Z_1$ und/oder $Z_2$ gleich C=O sind und Y, n, m, $R_1$, $R_2$ und $R_3$ die obigen Bedeutungen haben, zu einer Verbindung der Formel I, in welcher $Z_1$ und/oder $Z_2$ für CHOH stehen und Y, n, m, $R_1$, $R_2$ und $R_3$ die obigen Bedeutungen haben; oder
e) die Veresterung einer Verbindung der Formel I, in welcher $Z_1$ und/oder $Z_2$, gleich CHOH sind und Y, n, m, $R_1$, $R_2$ und $R_3$ die obigen Bedeutungen haben, zu einer Verbindung der Formel I, in welcher $Z_1$ und/oder $Z_2$ für

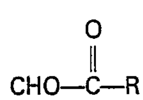

stehen und Y, n, m, $R_1$, $R_2$ und $R_3$ die obigen Bedeutungen haben; oder

f) die Hydrolyse einer Verbindung der Formel I, in welcher $Z_1$ und/oder $Z_2$ gleich $CHO-\overset{O}{\overset{\|}{C}}R$ sind und Y, n, m, $R_1$, $R_2$ und $R_3$ die obigen Bedeutungen haben, in eine Verbindung der Formel I, in welcher $Z_1$ und/oder $Z_2$ gleich CHOH sind und Y, n, m, $R_1$, $R_2$ und $R_3$ die obigen Bedeutungen haben; oder

g) die Umwandlung der freien Base der Verbindung der Formel I in ein pharmazeutisch annehmbares Säureadditionssalz; oder

h) die Umwandlung eines Salzes der Verbindung der Formel I in eine freie Base; oder

i) die Umwandlung eines Salzes der Verbindung der Formel I in eine anderes Salz.

6. Verfahren zur Herstellung einer pharmazeutischen Zusammensetzung nach Anspruch 4, in welchem die gemäß Anspruch 5 hergestellte wirksame Zutat mit einem pharmazeutisch annehmbaren Trägerstoff vermischt wird.

7. Verwendung einer Verbindung nach Anspruch 1, 2 oder 3 zur Herstellung einer pharmazeutischen Zusammensetzung.

**Patentansprüche für den Vertragsstaat: AT**

1. Verfahren zur Herstellung einer Verbindung der Formel I

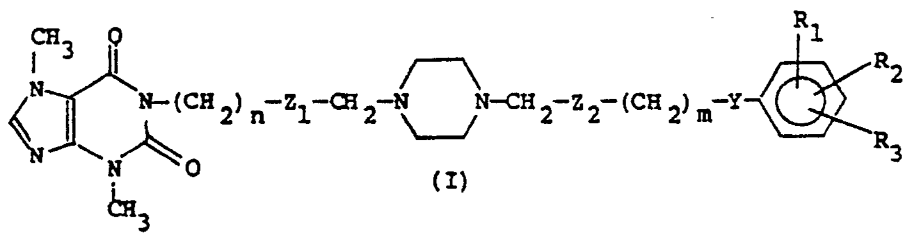

(I)

und der pharazeutisch annehmbaren Säureadditionssalze derselben, in welcher

$Z_1$ und $Z_2$ jeweils unabhängig voneinander ausgewählt sind aus der Gruppe bestehend aus $CH_2$, CHOB und C=O, wobei B ausgewählt ist aus der Gruppe bestehend aus Wasserstoff und

$$-\overset{O}{\overset{\|}{C}}-R$$

wobei R $C_{1-6}$ Alkyl bedeutet;

Y für Sauerstoff oder Schwefel steht;

n eine ganze Zahl von 0 bis 4 ist, aber nicht gleich 0 sein kann, wenn $Z_1$ gleich CHOB ist;

m eine ganze Zahl von 1 bis 4 ist; und

$R_1$, $R_2$ und $R_3$ jeweils unabhängig voneinander Wasserstoff, Halogen, Hydroxy, Trifluormethyl, $C_{1-6}$ Alkyl oder $C_{1-6}$ Alkoxy sind, welches umfaßt:

a) die Umsetzung einer Verbindung der Formel IIa

$$ThB-(CH_2)_n-Z_1-CH_2X \qquad (IIa)$$

oder IIb

$$ThB-(CH_2)_n-\underset{\diagdown\;O\;\diagup}{CH-CH_2} \qquad (IIb)$$

in welchen $Z_1$ und n wie oben definiert sind, mit einer Verbindung der Formel III

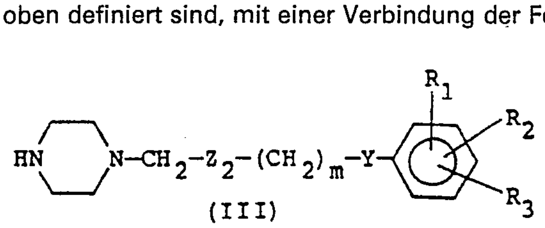

(III)

in welcher $Z_2$, m und Y wie oben definiert sind; oder

b) die Umsetzung einer Verbindung der Formel V

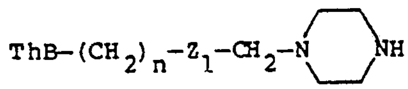

(V)

in welcher $Z_1$ und n wie oben definiert sind, mit einer Verbindung der Formel IVa

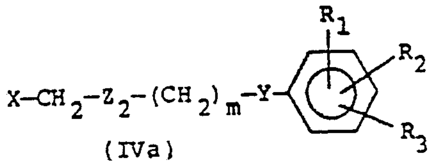

(IVa)

oder IVb

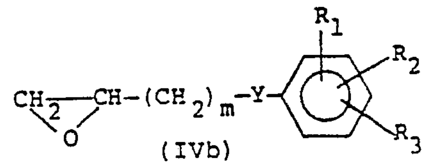

(IVb)

in welchen Z, m und Y wie oben definiert sind; oder

c) die Oxidation einer Verbindung der Formel I, in welcher $Z_1$ und/oder $Z_2$ CHOH sind und Y, n, m, $R_1$, $R_2$ und $R_3$ die obigen Bedeutungen haben, zu einer Verbindung der Formel I, in welcher $Z_1$ und/oder $Z_2$ C=O bedeuten und Y, n, m, $R_1$, $R_2$ und $R_3$ die obigen Bedeutungen haben; oder

d) die Reduktion einer Verbindung der Formel I, in welcher $Z_1$ und/oder $Z_2$ gleich C=O sind und Y, n, m, $R_1$, $R_2$ und $R_3$ die obigen Bedeutungen haben, zu einer Verbindung der Formel I, in welcher $Z_1$ und/oder $Z_2$ für CHOH stehen und Y, n, m, $R_1$, $R_2$ und $R_3$ die obigen Bedeutungen haben; oder

e) die Veresterung einer Verbindung der Formel I, in welcher $Z_1$ und/oder $Z_2$, gleich CHOH sind und Y, n, m, $R_1$, $R_2$ und $R_3$ die obigen Bedeutungen haben, zu einer Verbindung der Formel I, in welcher $Z_1$ und/oder $Z_2$ für

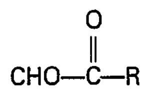

stehen und Y, n, m, $R_1$, $R_2$ und $R_3$ die obigen Bedeutungen haben; oder

f) die Hydrolyse einer Verbindung der Formel I, in welcher $Z_1$ und/oder $Z_2$ gleich 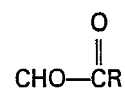

sind und Y, n, m, $R_1$, $R_2$ und $R_3$ die obigen Bedeutungen haben, in eine Verbindung der Formel I, in welcher $Z_1$ und/oder $Z_2$ gleich CHOH sind und Y, n, m, $R_1$, $R_2$ und $R_3$ die obigen Bedeutungen haben; oder

g) die Umwandlung der freien Base der Verbindung der Formel I in ein pharmazeutisch annehmbares Säureadditionssalz; oder

h) die Umwandlung eines Salzes der Verbindung der Formel I in eine freie Base; oder

i) die Umwandlung eines Salzes der Verbindung der Formel I in eine anderes Salz.

2. Verfahren nach Anspruch 1 zur Herstellung einer Verbindung der Formel I, in welcher $Z_1$ und $Z_2$ jeweils unabhängig voneinander $CH_2$ oder CHOH sind und m und n beide gleich 1 sind.

3. Verfahren gemäß Anspruch 1 zur Herstellung einer Verbindung der Formel I, nämlich 1-(3-Theobromin-1-yl-2-hydroxypropyl)-4-(3-phenylthiopropyl)piperazin.

4. Verfahren zur Herstellung einer pharmazeutischen Zusammensetzung, welches umfaßt das Vermischen einer wirksamen Menge einer wie in Anspruch 1 definierten Verbindung mit wenigstens einem pharmazeutisch annehmbaren Trägerstoff.

5. Verwendung einer wie in Anspruch 1 definierten Verbindung zur Herstellung einer pharmazeutischen Zusammensetzung.

**Revendications pour les Etats contractants: BE CH DE FR GB IT LI LU NL SE**

1. Composé de formule I

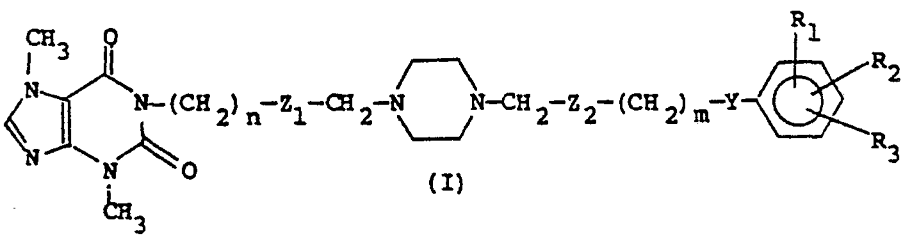

(I)

dans laquelle

$Z_1$ et $Z_2$ sont choisis chacun, indépendamment l'un de l'autre, parmi les groupes $CH_2$, CHOB et C=O, B représentant un atome d'hydrogène ou un radical

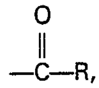

dans lequel R est un groupe alkyle en $C_{1-6}$;

Y est un atome d'oxygène ou de soufre;

n est un nombre entier allant de 0 à 4, mais ne peut pas être zéro lorsque $Z_1$ est CHOB;

m est un nombre entier allant de 1 à 4; et

$R_1$, $R_2$ et $R_3$ sont chacun, in dépendamment, un atome d'hydrogène ou d'halogène, ou un groupe hydroxy, trifluorométhyle, alkyle en $C_{1-6}$ ou alcoxy en $C_{1-6}$;

et sels d'addition d'acide pharmaceutiquement acceptables de celui-ci.

2. Composé selon la revendication 1, dans lequel

$Z_1$ et $Z_2$ son chacun, indépendamment l'un de l'autre, $CH_2$ ou CHOH; et

m et n sont chacun 1;

et sels d'addition d'acide pharmaceutiquement acceptables de celui-ci.

3. Composé selon la revendication 2, lequel est la 1-(3-théobromin-1-yl-2-hydroxypropyl)-4-(3-phénylthiopropyl)-pipérazine, et sels d'addition d'acide pharmaceutiquement acceptables de celui-ci.

4. Composition pharmaceutique comprenant une quantité efficace du composé de la revendication 1 ou un sel d'addition d'acide pharmaceutiquement acceptable de celui-ci en mélange avec au moins un excipient non toxique, pharmaceutiquement acceptable.

5. Procédé pour la préparation d'un composé selon la revendication 1, lequel procédé comprend

a) la réaction d'un composé de formule IIa

$$ThB—(CH_2)_n—Z_1—CH_2X \qquad (IIa)$$

ou IIb

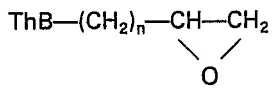

(IIb)

dans lesquelles $Z_1$ et n sont tels que définis plus haut, avec un composé de formule III

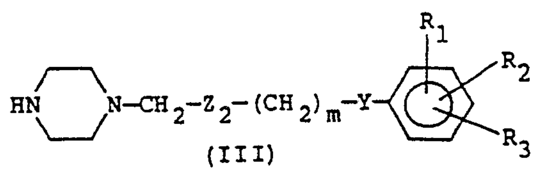

(III)

dans laquelle $Z_2$, m et Y sont tels que définis plus haut, ou

b) la réaction d'un composé de formule V

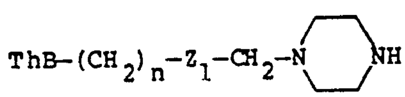

(V)

dans laquelle $Z_1$ et n sont tels que définis plus haut, avec un composé de formule IVa

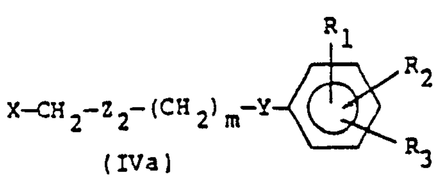

(IVa)

ou IVb

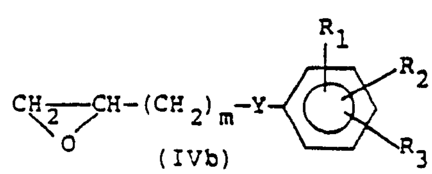

(IVb)

dans lesquelles Z, m et Y sont tels que définis plus haut; ou

(c) l'oxydation d'un composé de formule I dans lequel $Z_1$ et/our $Z_2$ représente(nt) CHOH, et Y, n, m, $R_1$, $R_2$ et $R_3$ ont les significations données plus haut, pour aboutir à un composé de formule I dans lequel $Z_1$ et/ou $Z_2$ représente(nt) C=O, et Y, n, m, $R_1$, $R_2$ et $R_3$ ont les significations données plus haut; ou

(d) la réduction d'un composé de formule I dans lequel $Z_1$ et/ou $Z_2$ représente(nt) C=O, et Y, n, m, $R_1$, $R_2$

et $R_3$ ont les significations données plus haut, pour aboutir à un composé de formule I dans lequel $Z_1$ et/ou $Z_2$ représente(nt) CHOH, et Y, n, m, $R_1$, $R_2$ et $R_3$ ont les significations données plus haut; ou

(e) l'estérification d'un composé de formule I dans lequel $Z_1$ et/ou $Z_2$ représente(nt) CHOH, et Y, n, m, $R_1$, $R_2$ et $R_3$ ont les significations données plus haut, pour aboutir à un composé de formule I dans lequel $Z_1$ et/ou $Z_2$ represente(nt)

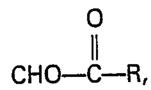

et Y, n, m, $R_1$, $R_2$ et $R_3$ ont les significations données plus haut; ou

(f) l'hydrolyse d'un composé de formule I dans lequel $Z_1$ et/ou $Z_2$ représente(nt) 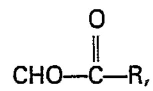

et Y, n, m, $R_1$, $R_2$ et $R_3$ ont les significations données plus haut, pour aboutir à un composé de formule I dans lequel $Z_1$ et/ou $Z_2$ represente(nt) CHOH, et Y, n, m, $R_1$, $R_2$ et $R_3$ ont les significations données plus haut; ou

(g) la conversion de la base libre du composé de formule I en un sel d'addition d'acide pharmaceutiquement acceptable; ou

(h) la conversion d'un sel du composé de formule I en une base libre; ou

(i) la conversion d'un sel du composé de formule I en un autre sel.

6. Procédé pour la préparation d'une composition pharmaceutique selon la revendication 4, dans lequel on mélange l'ingrédient actif préparé selon la revendication 5, avec un véhicule pharmaceutiquement acceptable.

7. Utilisation d'un composé selon la revendication 1, 2 ou 3, dans la préparation d'une composition pharmaceutique.

**Revendications pour l'Etat contractant: AT**

1. Procédé pour la préparation d'un composé de formule I

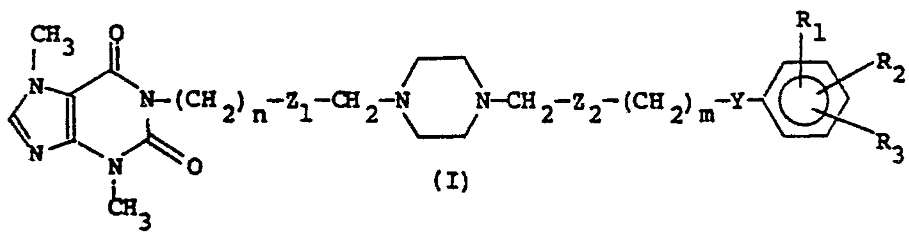

(I)

dans laquelle

$Z_1$ et $Z_2$ sont choisis chacun, indépendamment l'un de l'autre, parmi les groupes $CH_2$, CHOB et C=O, B représentant un atome d'hydrogène ou un radical

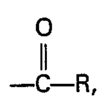

dans lequel R est un groupe alkyle en $C_{1-6}$;

Y est un atome d'oxygène ou de soufre;

n est un nombre entier allant de 0 à 4, mais ne peut pas être zéro lorsque $Z_1$ est CHOB;

m est un nombre entier allant de 1 à 4; et

$R_1$, $R_2$ et $R_3$ sont chacun, in dépendamment, un atome d'hydrogène ou d'halogène, ou un groupe hydroxy, trifluorométhyle, alkyle en $C_{1-6}$ ou alcoxy en $C_{1-6}$;

et des sels d'addition d'acide pharmaceutiquement acceptables de celui-ci,

lequel procédé comprend

a) la réaction d'un composé de formule IIa

$$ThB-(CH_2)_n-Z_1-CH_2X \qquad (IIa)$$

ou IIb

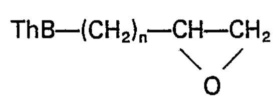

(IIb)

dans lesquelles $Z_1$ et n sont tels que définis plus haut, avec un composé de formule III

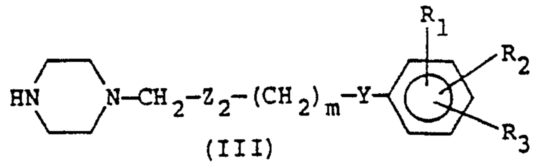

(III)

dans laquelle $Z_2$, m et Y sont tels que définis plus haut, ou

b) la réaction d'un composé de formule V

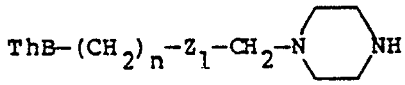

(V)

dans laquelle $Z_1$ et n sont tels que définis plus haut, avec un composé de formule IVa

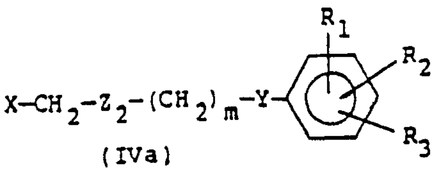

(IVa)

ou IVb

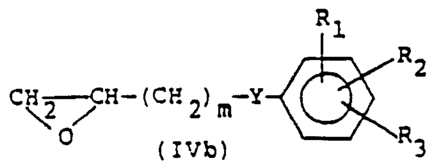

(IVb)

dans lesquelles Z, m et Y sont tels que définis plus haut; ou

(c) l'oxydation d'un composé de formule I dans lequel $Z_1$ et/ou $Z_2$ représente(nt) CHOH, et Y, n, m, $R_1$, $R_2$ et $R_3$ ont les significations données plus haut, pour aboutir à un composé de formule I dans lequel $Z_1$ et/ou $Z_2$ représente(nt) C=O, et Y, n, m, $R_1$, $R_2$ et $R_3$ ont les significations données plus haut; ou

(d) la réduction d'un composé de formule I dans lequel $Z_1$ et/ou $Z_2$ représente(nt) C=O, et Y, n, m, $R_1$, $R_2$ et $R_3$ ont les significations données plus haut, pour aboutir à un composé de formule I dans lequel $Z_1$ et/ou $Z_2$ représente(nt) CHOH, et Y, n, m, $R_1$, $R_2$ et $R_3$ ont les significations données plus haut; ou

(e) l'estérification d'un composé de formule I dans lequel $Z_1$ et/ou $Z_2$ représente(nt) CHOH, et Y, n, m, $R_1$, $R_2$ et $R_3$ ont les significations données plus haut, pour aboutir à un composé de formule I dans lequel $Z_1$ et/ou $Z_2$ represente(nt)

$$CHO-\overset{\overset{O}{\|}}{C}-R,$$

et Y, n, m, $R_1$, $R_2$ et $R_3$ ont les significations données plus haut; ou

(f) l'hydrolyse d'un composé de formule I dans lequel $Z_1$ et/ou $Z_2$ représente(nt) $CHO-\overset{\overset{O}{\|}}{C}-R$,

et Y, n, m, $R_1$, $R_2$ et $R_3$ ont les significaitons données plus haut, pour aboutir à un composé de formule I dans lequel $Z_1$ et/ou $Z_2$ represente(nt) CHOH, et Y, n, m, $R_1$, $R_2$ et $R_3$ ont les significations données plus haut; ou

(g) la conversion de la base libre du composé de formule I en un sel d'addition d'acide pharmaceutiquement acceptable; ou

(h) la conversion d'un sel du composé de formule I en une base libre; ou

(i) la conversion d'un sel du composé de formule I en un autre sel.

2. Procédé selon la revendication 1, pour la préparation d'un composé de formule I dans lequel $Z_1$ et $Z_2$ sont chacun, indépendamment l'un de l'autre, $CH_2$ ou CHOH, et m et n sont chacun 1.

3. Procédé selon la revendication 1, pour la préparation d'un composé de formule I, leque est la 1-(3-théobromin-1-yl-2-hydroxypropyl)-4-(3-phénylthiopropyl)-pipérazine.

4. Procédé pour la préparation d'une composition pharmaceutique, lequel comprend le mélange d'une quantité efficace d'un composé tel que défini dans la revendication 1, avec au moins un véhicule pharmaceutiquement acceptable.

5. Utilisation d'un composé tel que défini dans la revendication 1, dans la préparation d'une composition pharmaceutique.